# EUROPEAN PATENT APPLICATION

(11) **EP 4 325 514 A2**
(43) Date of publication of application: **21.02.2024**
(21) Application number: 23186417.4
(22) Date of filing: 19.07.2023
(51) Int. Cl.: G16H 30/40, G16H 20/40, A61B 5/02, A61B 18/00, A61B 5/00, A61B 18/14, A61B 8/00, A61B 5/107

(54) **MEASURING RENAL ARTERY DISTENSIBILITY AND/OR COMPLIANCE FROM MEDICAL IMAGES**

(30) Priority: 29.07.2022 US 202263393729 P; 16.06.2023 US 202318211070
(71) Applicant: Medtronic Ireland Manufacturing Unlimited Company, Dublin 2, D02 T380 (IE)
(72) Inventor: Coates, Paul J., Santa Rosa (US); Hettrick, Douglas A., Mounds View (US); Peterson, Darion R., Boulder (US)
(74) Representative: Zimmermann & Partner Patentanwälte mbB

(57) **Abstract**

A method of determining blood vessel stiffness including, acquiring a plurality of images of a portion of a blood vessel, analyzing the plurality of images to determine a maximum diameter of the portion of the blood vessel at a plurality of points along the blood vessel, analyzing the plurality of images to determine a minimum diameter of the portion of the blood vessel at the plurality of points along the blood vessel, determining a blood pressure experienced in the portion of the blood vessel, and calculating a stiffness of the portion of the blood vessel based on a ratio of the blood pressure and a difference in a diameter of the blood vessel at one or more of the plurality of points along the blood vessel.

## Description

### CROSS-REFERENCE TO RELATED APPLICATION

This application claims the benefit of, and priority to, U.S. Provisional Patent Application Serial No. 63/393,729, filed on July 29, 2022, the entire content of which is hereby incorporated by reference herein.

### TECHNICAL FIELD

The present technology relates generally to neuromodulation and associated systems and methods. In particular, the disclosure is directed to diagnostic methods and systems for pre-procedurally determining potential efficacy of neuromodulation therapy for a particular patient and peri-procedurally assessing the efficacy of a neuromodulation therapy.

### BACKGROUND

The sympathetic nervous system (SNS) is a primarily involuntary bodily control system typically associated with stress responses. Fibers of the SNS innervate tissue in almost every organ system of the human body and can affect characteristics such as pupil diameter, gut motility, and urinary output. Such regulation can have adaptive utility in maintaining homeostasis or in preparing the body for rapid response to environmental factors. Chronic activation of the SNS, however, is a common maladaptive response that can drive the progression of many disease states including hypertension, diabetes, and others. Excessive activation of the renal SNS has been identified in humans as a likely contributor to the complex pathophysiology of hypertension, states of volume overload (such as heart failure), and progressive renal disease. In a similar manner, excessive activation of the hepatic SNS has been identified with reduce liver glucose uptake leading to hyperglycemia among other conditions.

A variety of solutions have been proposed to address the over activity of the SNS. These solutions include pharmaceutical, surgical, and energy-based stimulation and denervation techniques. Of these solutions, the energy-based solutions including radiofrequency (RF), ultrasound, and cryogenic ablation have been shown to be effective in treating the disease states described above. The therapy is achieved by interrupting the SNS through denervation (e.g., severing the sympathetic nerves), without negatively impacting the function of the enervated organs (e.g., liver or kidneys) or the blood vessels along which the sympathetic nerves are found.

Despite the success of these therapies, not every patient suffering from these conditions will necessarily benefit from the therapies. Thus, improvements in determining the patient population that will benefit from these therapies and providing accurate pre-procedural expectations for both clinicians and patients is desired. In addition, improvements in peri-procedurally and post-procedurally assessing the success of the therapies are also desirable.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
Fig. 1 is a fluoroscopic image of a renal artery of a patient at a first time;
Fig. 2 is a fluoroscopic image of the renal artery of Fig. 1 at a second time;
Fig. 3 is a flow diagram of a method of measuring a body lumen wall stiffness and pulse wave velocity;
Fig. 4 is a fluoroscopic image of a renal artery with a denervation tool inserted therein in an undeployed state;
Fig. 5 is a fluoroscopic image of the body lumen and denervation tool of Fig. 5 in a deployed state;
Fig. 6 is a flow diagram of a method of measuring stiffness of the wall of the body lumen of Fig. 5;
Fig. 7 is a flow diagram of a method of determining whether a patient is likely to experience a positive outcome from a denervation procedure;
Fig. 8 is a schematic block diagram of a computing device in accordance with the disclosure; and
Fig. 9 is a flow diagram of a method of measuring stiffness of a body lumen wall in accordance with the disclosure.

### SUMMARY

This disclosure is directed to systems and methods of determining a stiffness of a body lumen wall (e.g., an artery). Further the disclosure is directed to methods and systems of assessing the stiffness to determine whether a patient will benefit from a denervation therapy.

One aspect of the disclosure is directed to a method of determining blood vessel stiffness, the method includes acquiring a plurality of images of a portion of a blood vessel. The method of determining blood vessel stiffness also includes analyzing the plurality of images to determine a maximum diameter of the portion of the blood vessel at a plurality of points along the blood vessel, analyzing the plurality of images to determine a minimum diameter of the portion of the blood vessel at the plurality of points along the blood vessel, determining a blood pressure experienced in the portion of the blood vessel. The method of determining blood vessel stiffness also includes calculating a stiffness of the portion of the blood vessel based on a ratio of the blood pressure and a difference in a diameter of the blood vessel at one or more of the plurality of points along the blood vessel. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The method further including calculating a pulse wave velocity of blood in the portion of the blood vessel based on a difference in time at which a maximum diameter is reached at two or more of the plurality of points along the blood vessel. The method further including identifying the plurality of points along the blood vessel; defining at each point a vector normal to a wall of the blood vessel; and measuring the maximum and minimum diameters along the vector between the point and an opposing point at which the vector intersects an opposite wall of the blood vessel. The method further including denervating at the portion of the blood vessel. In certain aspects, a reduction in stiffness in excess of a threshold value is indicative of a successful denervation. The portion of the blood vessel may be a portion of a renal artery, a hepatic artery, a splanchnic artery, a celiac artery, a superior mesenteric artery, or an inferior mesenteric artery, and successful denervation may result in a drop in the blood pressure of a patient. The second stiffness may be calculated peri-procedurally. In some aspects of the disclosure the portion of the blood vessel is a portion of a renal artery of a patient, and the calculated stiffness of the renal artery is indicative of a sympathetic nerve activity along the renal artery. In another aspect, the blood vessel identified for receiving denervation therapy is a portion of a renal artery of a patient, and the calculated stiffness of the renal artery is indicative of a sympathetic nerve activity along the renal artery. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

A further aspect of the disclosure is directed to a method of assessing a stiffness of a blood vessel, the method includes inserting an elongate tool into a blood vessel. The method also includes applying a known force to a wall of the blood vessel using the elongate tool; capturing a plurality of images of the blood vessel, measuring in the plurality of images a change in diameter of the blood vessel caused by application of the known force by the elongate tool to the wall of the blood vessel, and calculating a stiffness of the blood vessel based on a ratio of the known force and the measured change in diameter of the blood vessel. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The method where the blood vessel is a portion of a renal artery of a patient, and the calculated stiffness of the renal artery is indicative of a sympathetic nerve activity along the renal artery. The elongate tool may be a denervation tool. A reduction in stiffness in excess of a threshold value may be indicative of a successful denervation. The second stiffness may be calculated peri-procedurally. The method may further include denervating at the renal artery. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

Still a further aspect of the disclosure is directed to a method of assessing stiffness of a blood vessel. The method of assessing stiffness includes measuring a blood pressure of a patient. The method of assessing stiffness also includes calculating a stiffness of a blood vessel identified for receiving denervation therapy; normalizing the calculated stiffness of the blood vessel relative to the measured blood pressure of the patient, and determining that the patient is likely to experience a positive response to a denervation when the normalized stiffness is greater than a predetermined value. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The method further including measuring a mean aortic pressure (MAP) of the patient and comparing the measured MAP and calculated stiffness to a database of MAP and stiffness data to determine the normalized stiffness of the blood vessel. The method further including comparing the calculated stiffness and measured blood pressure of the blood vessel to known tissue properties at a reference blood pressure to determine the normalized stiffness of the blood vessel. The method further including measuring a blood pressure of a plurality of patients; calculating a stiffness of a blood vessel for the plurality of patients; and correlating the measured blood pressure and calculated blood vessel stiffness of the plurality of patients to an expected outcome of denervation of the blood vessel. Normalizing the calculated stiffness may further include applying a stimulus to the patient and calculating an after-stimulus stiffness of the blood vessel, where a response to the stimulus is indicative of a likelihood of a patient experiencing a positive response to a denervation of the blood vessel. The stimulus may be a non-invasive stimulus including one or more of a Valsalva maneuver, Müller's maneuver, cold pressor, or a mental stress test. The stimulus may be an invasive stimulus including one or more of an application of a vasodilator or a vasoconstrictor. The method may further include denervating at the blood vessel. A reduction in stiffness in excess of a predetermined value may be indicative of a successful denervation. The blood vessel may be one or more a renal artery, a hepatic artery, a splanchnic artery, a celiac artery, a superior mesenteric artery, or an inferior mesenteric artery. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

Yet a further aspect of the disclosure is directed to a method of assessing a stiffness of a blood vessel. The method includes measuring a blood pressure of a patient. The method also includes reducing a local blood pressure in the blood vessel identified for receiving denervation therapy relative to the measured blood pressure; capturing a plurality of images of the blood vessel identified for receiving denervation therapy, measuring a diameter of the blood vessel identified for receiving denervation therapy at a plurality of points in each of the plurality of images, calculating a stiffness of the blood vessel identified for receiving denervation therapy from changes in measured diameter of the blood vessel at the plurality of points, and determining that the patient is likely to experience a positive response to a denervation when the calculated stiffness is greater than a predetermined value. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The method further including, analyzing the plurality of images to determine a maximum diameter of the blood vessel identified for receiving denervation therapy at the plurality of points along the blood vessel. The method further including, analyzing the plurality of images to determine a minimum diameter of the blood vessel identified for receiving denervation therapy at the plurality of points along the blood vessel. Calculating the stiffness the blood vessel identified for receiving denervation therapy may be based on a ratio of the local blood pressure and a difference in a diameter of the blood vessel at one or more of the plurality of points along the blood vessel. The method further including defining at each point a vector normal to a wall of the blood vessel; and measuring the maximum and minimum diameters along the vector between the point and an opposing point at which the vector intersects an opposite wall of the blood vessel. The method further including denervating a portion of the blood vessel identified for receiving denervation therapy. A reduction in stiffness in excess of a threshold value may be indicative of a successful denervation. The blood vessel identified for receiving denervation therapy may be a portion of a renal artery, a hepatic artery, a splanchnic artery, a celiac artery, a superior mesenteric artery, or an inferior mesenteric artery, and successful denervation results in a drop in the blood pressure of a patient. The second stiffness ma be calculated peri-procedurally. The method further including calculating a pulse wave velocity of blood in the blood vessel identified for receiving denervation therapy based on a difference in time at which a maximum diameter is reached at two or more of the plurality of points along the blood vessel identified for receiving denervation therapy. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

Further disclosed herein is a method of determining blood vessel stiffness including, acquiring a plurality of images of a portion of a blood vessel, analyzing the plurality of images to determine a maximum diameter of the portion of the blood vessel at a plurality of points along the blood vessel, analyzing the plurality of images to determine a minimum diameter of the portion of the blood vessel at the plurality of points along the blood vessel, determining a blood pressure experienced in the portion of the blood vessel, and calculating a stiffness of the portion of the blood vessel based on a ratio of the blood pressure and a difference in a diameter of the blood vessel at one or more of the plurality of points along the blood vessel.

### DETAILED DESCRIPTION

This disclosure is directed to methods and systems for assessing the likelihood that a patient is going to respond to sympathetic nerve system (SNS) denervation. Further the disclosure is directed to systems and methods of providing feedback to clinicians during a therapy indicating the effective SNS denervation.

It has been observed that vascular mechanical stiffness can be an indicator of a likelihood of successful denervation procedures. Stiffness is, at least in part, attributable to an overstimulation of the SNS, the sympathetic nerves running along an artery, and their stimulation causes smooth muscle of the artery to contract making the blood vessel stiff. One currently used indicator of compliance or stiffness is arterial pulse wave velocity (PWV). In general, greater stiffness is associated with higher PWV and lesser stiffness with lower PWV. Current measurement techniques of PWV often require additional and invasive hardware to undertake the procedure (e.g., pressure wires, flow wires, combo wires). These hardware devices must be placed within the patient in order measure the PWV within the desired artery, further the presences of these devices alone can lead to inaccuracies in PWV measurement based their changing of the local conditions within the artery. In addition, to be effective in their measurement, these hardware devices may further require the use of one or more medications such as adenosine. As such these hardware devices, to the extent they are deemed effective in measuring PWV, are better suited for periprocedural use to provide some form of feedback to the clinician on the efficacy of a therapy (e.g., change in PWV because of the application of therapy).

The stiffness of the arterial wall is the result of multiple factors including the mechanical properties and tone of the smooth muscle, collagen, and elastin content of the arterial wall and the adventitia. Other factors include features within or about the artery in question such as arterial disease, plaque, and fibromuscular dysplasia, among others. Thus, stiffness is a complex attribute of a patient's arteries having many components and PWV is merely a derivative indicator of that stiffness.

PWV is not a direct measurement of the stiffness and PWV measurements provide only a proxy measurement that consider the local mechanics of every smooth muscle cell, plaque, and other physiological and disease state factors to provide an indicator of average/integrated wall stiffness over any given region. Further, there are challenges in PWV measurement that not only make PWV measurement ill-suited for pre-procedural assessment even with the tools described above, but also make it an inexact indicator of therapy success. One such issue is the presence of reflected PWV as a result of arterial bends. The reflected PWV can result in inexact measurements. As a result, even where measured using current devices, changes in PWV are not necessarily an indicator of procedural success.

Thus, there is a need for a more direct mechanism for measurement of arterial stiffness that overcomes the shortcomings of current PWV measurement techniques. Further there is a need for methods and systems that enable preprocedural assessments without requiring the invasive insertion of one or more devices into the patient.

As noted above one of the effects of hyperactivity of the SNS is for the arteries to lose flexibility (i.e., become more stiff). By having the nerves constantly signaling, the arterial smooth muscle cells are never allowed to fully relax, resulting in heightened stiffness of the artery wall. As explained in greater detail below, the smooth muscle tone is one factor of arterial stiffness that can have its mechanical properties changed over a short time-period (e.g., by RF denervation). Thus, RF denervation of sympathetic nerves, in for example, the renal or hepatic arteries of a patient results in relaxation of the smooth muscles of the arterial wall and therewith an observable change in stiffness of the artery. Accordingly, this disclosure is directed to methods and systems of assessing arterial stiffness, determining the likelihood of a denervation procedure resulting in a change of arterial stiffness, and a feedback mechanism for clinicians indicating a successful denervation has been achieved because of a change in arterial stiffness.

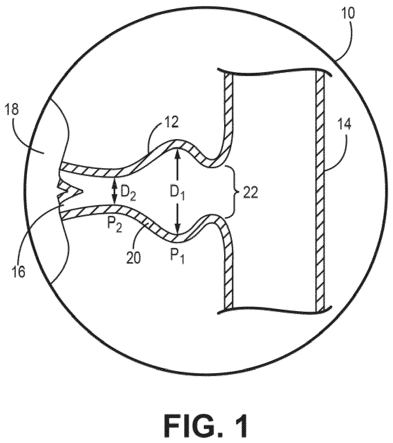

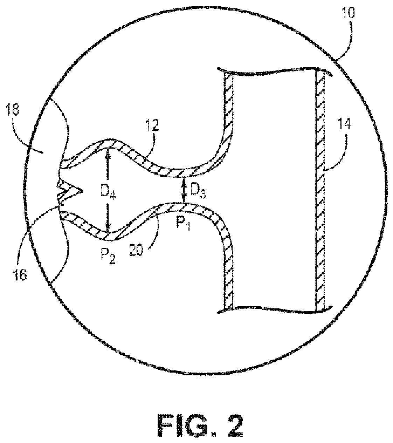

Some aspects of the disclosure are directed to assessing arterial stiffness from medical images. Although some examples are disclosed with reference to fluoroscopic images and fluoroscopic imaging devices, images and devices of other imaging modalities also can be used to observe the blood vessel or any body lumen to undertake the analyses disclosed herein. As is known, fluoroscopy and contrast enhanced fluoroscopy are tools used in a variety of medical procedures to non-invasively observe the organs and other tissues of a patient. In accordance with the disclosure an assessment of the stiffness of renal arteries is depicted in Figs. 1 and 2. Fig. 1 depicts a fluoroscopic image 10 of the renal artery 12 as it descends from the aorta 14 and the renal artery branches 16 before entering the kidney 18. The fluoroscopic image 10 is taken at a time t1. At time t1 at a first point P1 along the renal artery a diameter D1 of the renal artery is measured. Also at time t1, a second diameter D2 is measured at point P2.

Each cardiac cycle, about 10% of the cardiac output travels through each kidney 18. As a result, systole there is significant outward pressure on the renal artery 12, and particularly the arterial wall 20 that goes away during diastole. If the renal artery and its branches 16 are very stiff, there will be little or no change in diameter across a cardiac cycle, but if the arteries are normal there will be a measurable diameter change.

Fig. 2 depicts a fluoroscopic image of the renal artery 12 at a time t2. At point P1, the renal artery 12 has a diameter D3 and a diameter at point P2 of D4. As will be appreciated, the differences in diameter D1 to D3 and D2 to D4 are exaggerated in Figs. 1 and 2 to promote an understanding of the parameters being measured. The comparison of D1 to D3 and D2 to D4 demonstrate a change in diameter of the renal artery 12 at points P1 and P2 caused by the cardiac cycle. Blood vessel strain can thus be described as the for example, D4-D2/D2, this may also be described herein as the distensibility of the blood vessel. The fluoroscopic images 10 of Figs. 1 and 2 may be acquired at a frequency of 5 Hz, 10 Hz, 15, Hz, 20 Hz, 25 Hz, 30 Hz or higher frequencies (and frequencies between any of these listed frequencies) to provide sufficient resolution. Further, the use of contrast medium injected into the blood stream of the patient, helps improve the resolution of the boundary between the arterial wall 20 and the blood carried through the blood vessel.

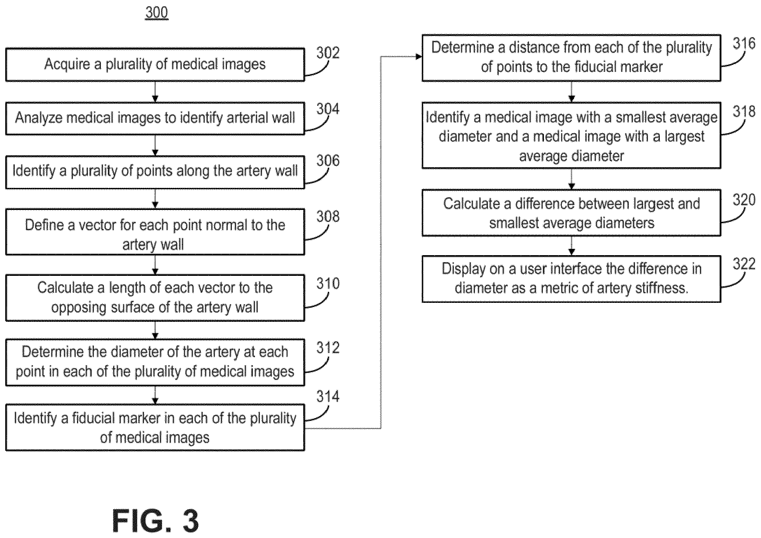

One method of employing the fluoroscopic images 10, as depicted in Figs. 1 and 2, to determine the stiffness of the arteries of a patient, particularly the renal or hepatic arteries is described in connection with Fig. 3. In accordance with the method 300, a series of fluoroscopic images 10 of a renal artery of a patient is acquired at step 302. At step 304, the acquired fluoroscopic images 10 are analyzed, for example, by an automatic or computerized image analysis software stored in a memory and executed by a processor on a computer, laptop or tablet, to distinguish the artery wall 20 from the blood flowing through the artery 12. In one aspect of the disclosure an image is analyzed to identify a pixel density or brightness change (measured typically in Hounsfield units) from the dark areas of the contrast rich blood to the lighter areas identifying the boundary of the arterial wall 20. Once the arterial wall 20 is defined, at step 306 a plurality of points (e.g., P1 and P2 of Figs. 1 and 2) are identified along the arterial wall 20 of the renal artery 12. For each of the plurality of points identified at step 308 a vector normal to the arterial wall 20 at the point P1 or P2 is directed towards the arterial wall 20 opposite the identified point. The intersection of the vector and an opposite surface of the arterial wall 20 defines a length that is the diameter of the renal artery 12 (e.g., D1, D2, D3, D4) and is calculated at step 310. For each image of the series of images, the diameter at each of the plurality of points along the renal artery 12 is calculated at step 312.

As will be appreciated each fluoroscopic image of the series of fluoroscopic images is acquired at different times, for example, a system operating at 10 Hz will generate 10 images per second or one every 1/10^{th} of a second. Thus, each diameter calculated at step 312 is associated with a particular time (e.g., t1, t2, etc.).

At step 314 a fiducial marker is identified in the plurality of fluoroscopic images. The fluoroscopic marker may be for example the ostium 22 of the renal artery 12 from the aorta 14 identified based on the change in direction of the contrast from the aorta 14 to the renal artery 12. Though other natural and manufactured fiducial markers may also be employed without departing from the scope of the disclosure. At step 316, a distance of each of the plurality of points (e.g., P1, P2) at which the diameters (e.g., D1, D2, D3, D4) are calculated can be measured from the fiducial marker (e.g., as an arc length). The result is a data set identifying a diameter of each point and its distance from the fiducial marker at each time (t1, t2) during the acquisition of the plurality of fluoroscopic images.

At step 318 the data set is analyzed to identify an image with a smallest average blood vessel diameter and an image with a largest average blood vessel diameter. These two images correlate to the point in the cardiac cycle with the lowest renal blood flow and the largest renal blood flow, respectively. At step 320 the smallest average diameter and the largest average diameter are compared to calculate a diameter difference either as an absolute or as a percentage. That comparison is a metric of vessel stiffness and can be output to a user at step 322 (e.g., via a user interface on a computer tasked with analyzing the images). Specifically, the metric of stiffness is the strain or distension, or the blood vessel caused by the cardiac cycle.

At step 322, displaying the difference in diameter, need not display an actual measurement, rather consistent with the disclosure herein the user interface may simply display an indication that the artery has a stiffness in excess of a threshold. That threshold may be indicative that the patient is likely to respond to the denervation therapy. As such the indication may be as simple as a green marker being displayed on the user interface. Alternatively, if the artery is deemed not to have a stiffness in excess of a threshold a red indicator may be displayed on the user interface indicating that the patient is unlikely to respond to denervation therapy.

The method 300 can be performed pre-procedurally, peri-procedurally, or post procedurally. In this manner the same method may be employed to assess the pre-treatment stiffness of the artery, the stiffness of the artery during the procedure, and to assess the post therapy stiffness of the artery. Further details of this may be observed in conjunction with the methods 600 and 700, discussed herein below. As will be appreciated, the indicator may take nearly any form including a text based indicator, color based indicator, shape based indicator, combinations of each of these (e.g., a red octagonal shape with the text "Non-responder"), etc., to inform the clinician as to the relative stiffness of the artery and therewith the likelihood that the patient is likely to respond to the therapy.

In some instances, the measurement of a strain or distension lower that a threshold value, indicative of a stiff blood vessel, may be sufficient to determine whether the patient is likely to respond to a denervation procedure. However, additional factors may need to be consider as described below.

Another aspect of the disclosure is directed to a method of calculating PWV using the fluoroscopic images captured at step 302. As will be appreciated, the minimum diameters (D3, D2) at two points (P1, P2) and the maximum diameters (D 1, D4) at two points (P1, P2) of Figs. 1 and 2 will not occur at the same times but rather occur at times t1 and t2. This is in part due to the systole blood pulse arriving sooner proximately (P1) and later distally (P2). Accordingly, in addition to measuring stiffness as described above in connection with method 300, at step 324 a duration from t1 to t2 for points P1 and P2 to achieve their largest diameters (D1 and D4) and the calculated arc-length between P1 and P2 can be utilized to estimate the PWV thorough the artery. The PWV is the distance between P1 and P2 and the time that elapses between the occurrence of D1 at point P1 and the occurrence of D4 at point P2. The same calculation can be made comparing the occurrence of D2 and D3. That is, the PWV can be based on the diameter maximum occurrence times or the minimum occurrence times. Either of these may be utilized and averaged over a period of time to provide an average PWV for the artery in question (e.g., the renal or hepatic artery). As described above, PWV provides a secondary indication of the stiffness of the artery. Unlike the techniques described above, the PWV calculated from non-invasively collected data provide another indication of the stiffness of the artery in question that may be used alone or in combination with the stiffness data calculated based on the change in diameter of the artery described above.

Though described herein as generally directed to assessing the renal arteries of a patient including its branches, the application is not so limited. The methods and systems described herein may be employed to assess the stiffness of any blood vessel including without limitation the hepatic arteries including the common hepatic artery, proper hepatic artery, and branches of the hepatic artery. Further the systems and methods may be employed to assess the stiffness splanchnic arteries, the celiac artery, the superior mesenteric artery, the inferior mesenteric artery and combinations of these arteries.

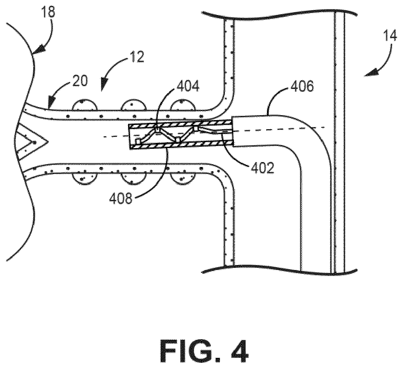

While the above method 300 is generally non-invasive (other than the application of the contrast medium) and could be performed both pre-procedurally and peri-procedurally, the instant disclosure is not so limited. In accordance with the disclosure a denervation tool 402 is shown in Fig, 4 in a pre-deployed state in a renal artery. In this example the denervation tool 402 is an elongate tool having a shape memory distal portion 404 having a helically shaped multi-electrode configuration. As shown in Fig. 4, the denervation tool 402 is within a catheter 406 which restrains a shape memory distal portion 404 to the diameter of the catheter 406 allowing the denervation tool 402 and catheter 406 to be navigated to a desired location within the anatomy of a patient (here a renal artery 12 proximate the kidneys 18 of a patient).

Those of ordinary skill in the art will recognize that a variety of navigation devices may be employed for inserting the catheter 406 and denervation tool 402 into the renal artery or other desired blood vessel, and even other body lumens to address for example stiffness of the wall of the renal pelvis with access through the ureter. These navigation devices include a guidewire (not shown) and an articulating mechanism on the catheter 406 enabling deformation of a distal portion of the catheter 406 to allow the catheter 406 to be shaped to advance from the aorta 14 and into the renal artery 20. Further, the articulation may employ one or more pull wires affixed to a pull ring formed in a distal portion of a catheter 406 and an actuator on a proximal end of the catheter 406 configured for actuation by a user to alter the shape of the catheter 406. The catheter 406 may also be robotically driven via a user interface allowing the clinician to manipulate the catheter 406 robotically.

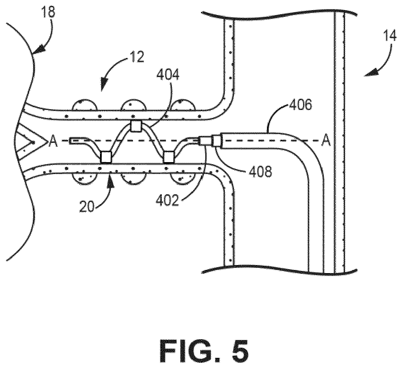

In Fig. 5, a sleeve 408 associated with the catheter 406 has been retracted exposing the shape memory distal portion 404 of the denervation tool 402 and allowing the shape memory distal portion 404 to expand to the size of the renal artery 12. The shape memory distal portion 404, upon being released from the catheter 406 applies a known force to the arterial wall 20 in an attempt to regain its unconstrained shape. This known force is balanced by an inwards force exerted by the arterial wall 20. In order to exert an incremental inwards force on the shape memory distal portion 404 the arterial wall 20 deforms and the degree of deformation provides information and vessel stiffness. If the blood vessel is stiff, it will deform less to achieve the required inwards force. If the blood vessel is not stiff, the blood vessel will deform more in order to achieve the required inward force. Therefore, an evaluation of blood vessel changes in diameter upon deployment of the shape memory distal portion 404 provides a measure of blood vessel stiffness.

Those of skill in the art will recognize that though described in connection with a shape memory distal portion 404, the denervation tool 402 is not so limited and may be comprised of any design that can apply a known force on the arterial wall 20 to deform the diameter of the arterial wall. For example, one or more balloons, palpation ends, or other configurations of the denervation tool or a separate palpation device formed as part of an elongate tool may be employed without departing from the scope of the disclosure in order to palpate or apply force to the wall 20 of the blood vessel to observe the deformation of the blood vessel. Further, though the denervation tool 402 as described herein above is a multi-electrode device as might be used in connection with a radio-frequency (RF) denervation catheter, the instant disclosure is not so limited. The denervation tool 402 may be of any modality including without limitation a bipolar or monopolar RF, microwave, cryoablation, ultrasound ablation and others without departing from the scope of the disclosure.

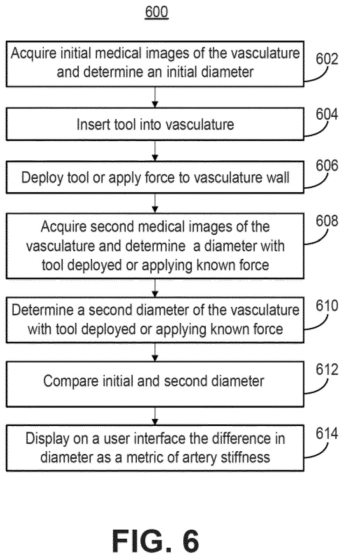

A method 600, outlined in Fig. 6, describes the measurement of the stiffness of a blood vessel, such as the renal artery, utilizing the denervation tool 402 and its deployment as shown in Figs. 4 and 5. At step 602 initial fluoroscopic images of the relevant vasculature of the patient is acquired and a diameter of the relevant vasculature is measured. For example, the automated image analysis software may be employed as described above to determine the diameter of the vasculature where the denervation tool 402 is to be deployed. At step 604 a denervation tool 402 or a palpation tool is navigated to a desired location in the vasculature of the patient. At step 606 a shape memory distal portion 404 of the denervation tool 402 is deployed from the catheter 406 and a known force is applied to the arterial wall 20. Alternatively, at step 606 the palpation device may be deployed to apply a known force to the arterial wall 20. At step 608, a second fluoroscopic image is acquired. A second diameter of the arterial wall 20 caused by the arterial wall's reaction to the application of the known force of the shape memory distal portion 404 is measured at step 610 in the second fluoroscopic image. In accordance with a further aspect of the disclosure, steps 606 and 610 may be undertaken multiple times in succession so that a dynamic response to the application of force can be observed (e.g., as in a fluoroscopic video), such dynamic applications and observations may provide further clarity on the stiffness of the blood vessel as not only will the deformation of the blood vessel be observable, but also the return of the blood vessel to its unstressed position.

Additionally or alternatively, rather than measure the displacement of the arterial wall 20, the dimension of the shape memory distal portion 404 after deployment may be measured to determine the displacement of the arterial wall 20 caused by the application of force by the shape memory distal portion 404. The ultimate shape of the distal portion 404 upon deployment in reaction to contact with the vessel wall can be measured and observed and the degree of deformation can be correlated to vessel stiffness. As will be apparent to those of skill in the art distal portion 404 may be easier to resolve under imaging modalities such as fluoroscopy. In at least one aspect the shape memory distal portion 404 is formed of a highly visible material having a specific and pre-defined degree of deformation based that provides an indication of vessel stiffness. Again, the image analysis methods described above in connection with method 300 may also be employed here to determine a change in diameter of the artery wall 20 or the shape memory distal portion 404 caused by the known force.

As with method 300, the determined diameter of the relevant vasculature from step 602 and from step 610 are compared at step 612 to calculate a diameter difference (either as an absolute value or a percentage). That comparison is a metric of vessel stiffness and can be output to a user at step 614 (e.g., via a user interface on a computer tasked with analyzing the fluoroscopic images).

As with step 322, described above, displaying the difference in diameter in connection with step 614, need not display an actual measurement, rather consistent with the disclosure herein the user interface may simply display an indication that the artery has a stiffness in excess of a threshold. That threshold may be indicative that the patient is likely to respond to the denervation therapy. As such the indication may be as simple as a green marker being displayed on the user interface. Alternatively, if the artery is deemed not to have a stiffness in excess of a threshold a red indicator may be displayed on the user interface indicating that the patient is unlikely to respond to denervation therapy.

The method 600 can be performed pre-procedurally, peri-procedurally, or post procedurally. In this manner the same method may be employed to assess the pre-treatment stiffness of the artery, the stiffness of the artery during the procedure, and to assess the post therapy stiffness of the artery. Further details of this may be observed in conjunction with the method 700, discussed herein below. As will be appreciated, the indicator may take nearly any form including a text-based indicator, color-based indicator, shape-based indicator, combinations of each of these (e.g., a red octagonal shape with the text "Non-responder"), etc., to inform the clinician as to the relative stiffness of the artery and therewith the likelihood that the patient is likely to respond to the therapy. Further, artery stiffness may be correlated to predict the response. The measured stiffness may inform the clinician of a predicted reduction in blood pressure or a range or predicted responses in mm/Hg as a result of the measured stiffness.

As will be appreciated the metric of vessel stiffness output at steps 322 and 614 of methods 300 and 600, respectively, are determinations of the amount of distension of a blood vessel such as the renal artery experiences during the cardiac cycle or by application of a known force, these are measurements of strain (a comparison of the change in diameter of the blood vessel). However, actual stiffness of a blood vessel is a comparison of the stress applied to the blood vessel compared to strain (distension of the blood vessel) that the stress achieves. Thus, in the example of method 600, the stiffness of the blood vessel would be characterized as the value of the known force/the change in diameter of the artery wall caused by the known force. Complicating matters slightly for blood vessel assessment, is the fact that arterial smooth muscle has non-Hookean mechanical properties. That is, the arterial smooth muscle exhibits a non-linear stress-strain curve. Accordingly, a blood vessel generally gets stiffer as it distends. Thus, distensibility of a blood vessel as determined in methods 300 and 600, when consider alone do not necessarily provide a complete indication of the stiffness of a blood vessel as both determinations ignore the effects of blood pressure in the blood vessel being measured. They are nonetheless informative, but a complete assessment of stiffness requires normalization with respect to the blood pressure of the patient.

In a further aspect of the disclosure, the distensibility data of a blood vessel collected in methods 300 and 600 are normalized based on the patient's blood pressure to calculate an actual stiffness of the blood vessel. As a general proposition a patient with high blood pressure will generally appear to have stiffer arteries than that same patient if distensibility could be measured at lower blood pressure. In many patients with high average blood pressure, the blood pressure pushes out on the vessel wall 20 and reduces the ability of the blood vessel to change diameter as a result of the cardiac cycle as described in method 300 or by the direct application of force described in method 600 because of this increased stiffness caused by the blood pressure.

In accordance with one aspect of the disclosure a mean aortic pressure (MAP), which is typically measured in interventional procedures that use arterial access, is determined for the patient either before or during the procedure. The MAP can then be employed to normalize the measured distensibility of the blood vessel (e.g., from methods 300 and 600) by adjusting measured distensibility by an index value. The index value may be calculated by measuring arterial distensibility in a population of patients and determining the correlation between MAP and distensibility. This population of patients would have patient's expressing as many levels of blood pressure as possible in an effort to correlate the effects of blood pressure, particularly high blood pressure on measured distensibility of the patient's blood vessels, particularly the arteries. Additionally or alternately, the index value can be determined computationally based on known tissue properties of the blood vessel and calculating distensibility at a reference pressure given measured distensibility at actual pressure. For patients with high blood pressure, the actual distensibility would be expected to be greater than the measured distensibility. In yet another aspect of the disclosure, to directly assess the distensibility of an artery of a patient having high blood pressure, a blood pressure regulating device (e.g., a clamp that reduces the effective diameter of a blood vessel or blood vessel occluding device that reduces the flow volume) may be temporarily applied to, for example, the renal artery. The regulating device reduces the pressure in the renal artery relative to aortic pressure. The pressure regulating device may be placed at or near the renal ostium and employed to throttle blood flow into the renal artery which lowers the blood pressure in the renal artery (or another blood vessel being assessed) relative to MAP. In this manner, an accurate assessment of the actual distensibility of a patient's blood vessel, even a patient having high blood pressure, can be undertaken using methods 300 or 600 described above and the distensibility normalized by the blood pressure provides an accurate assessment of the stiffness of the blood vessel. This normalization reduces the effects of the blood pressure on the calculated distensibility and provides a more accurate assessment of the stiffness of the blood vessel and the likelihood that the blood vessel's stiffness is the result of overactivation of the sympathetic nerves that run along the blood vessel.

A further aspect of the disclosure is a method of categorizing potential patients as likely to benefit from a denervation procedure based on the calculated stiffness of for example the renal or hepatic arteries. In accordance with this aspect of the disclosure data is collected from a plurality of patients. These patients include patients with normal blood pressure, high blood pressure, low blood pressure. The distensibility of the blood vessels for each patient is measured and an actual stiffness calculated. The patients then undergo a denervation procedure to denervate the sympathetic nerves surrounding the blood vessel whose stiffness has been measured. The patients are then observed for a change in stiffness of the blood vessel after the denervation process. The data are then categorized to identify a group as "Responders" to denervation therapy and "Non-responders" to denervation therapy. A threshold or predetermined pre-procedural stiffness can then be established for the assessment of new patients. A pre-procedurally determined "Responder" may be defined as having a distensibility or stiffness consistent with the responder group (artery determined to have a stiffness greater than the threshold or predetermined value) and a pre-procedurally determined "Non-responder" may be defined as having a distensibility or stiffness consistent with the non-responder group (artery determined to be flexible and have a stiffness less than a threshold or predetermined value).

Further, the data can also provide a range of expected outcomes and benefits from a denervation therapy based on the pre-procedurally measured stiffness of the blood vessel. Thus, both patient and clinician expectations for the efficacy of the therapy may be appropriately managed and for borderline cases the clinician can more accurately weigh the expected benefits to the patient vs. any potential for complications or benefits of alternative therapies. Further, these expected outcomes can be compared against observed outcomes, which may provide an assessment of the performance of the denervation procedure.

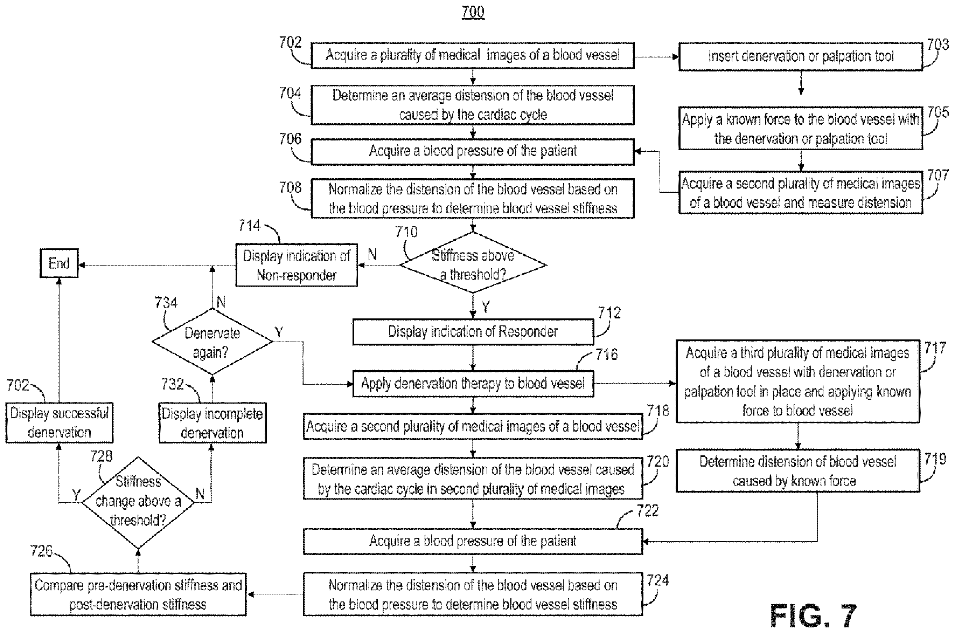

Accordingly, Fig. 7 describes a method 700 for determining whether a particular patient is likely to benefit from a denervation procedure. At step 702 a plurality of fluoroscopic images is acquired of a blood vessel. At step 704 an average distension of the blood vessel caused by the cardiac cycle at a plurality of points is determined, for example by the method 300 described above. The blood pressure of the patient is acquired at step 706. The distension of the blood vessel is normalized at step 708 based on the blood pressure to determine the stiffness of the blood vessel. The determined stiffness from step 708 is then compared to a threshold at step 710. If the determined stiffness is above a threshold a user interface on a computer tasked with analyzing the fluoroscopic images outputs an indication that the patient is likely to respond to denervation therapy at step 712. If the determined stiffness is below a threshold the user interface outputs an indication that the patient is unlikely to respond to denervation therapy at step 714.

At step 716 a denervation therapy is applied to the patient to denervate sympathetic nerves along the blood vessel whose stiffness was measured. Following denervation, a second series of fluoroscopic images are acquired at step 718. At step 720 an average distension of the blood vessel is again determined in the same manner as at step 704. The blood pressure of the patient is again acquired following the denervation at step 722. The measured distension of the blood vessel is normalized at step 724 based the blood pressure as described above at step 708 to determine the after-denervation stiffness of the blood vessel. At step 726 a comparison of the stiffness of the blood vessel before denervation (Step 708) to the stiffness after denervation (step 722) is undertaken. Where the change in stiffness exceeds a threshold value, the denervation is deemed successful at step 728 the user interface may display an indication of success at step 730 and the procedure ends. If the change in stiffness does not exceed a threshold the user interface associated with the computer performing the image analysis and stiffness determinations may display an indication that the denervation is incomplete at step 732 and inquire whether the user would like to attempt another denervation at step 734. If yes, the process returns to step 716, if not the method ends.

Alternatively, following acquisition of the fluoroscopic images at step 702, a denervation tool or palpation device is inserted into the blood vessel at step 703. At step 705 the denervation tool or palpation device applies a known force to the blood vessel wall. A second set of fluoroscopic images is acquired at step 707 and the distension of the blood vessel is measured by the image processing software described above running a computer as described above with respect to method 600. At this point the alternative process returns to step 706 and continues to step 718. At step 717 in accordance with the alternative process the acquired fluoroscopic images are a third set of fluoroscopic images that is acquired with the denervation or palpation tool in the blood vessel and in the extended state so that the post-denervation distension of the blood vessel can be analyzed. The distension of the blood vessel is calculated at step 719 in accordance with the methods described above in method 600. Following calculation of the post-denervation distension, the method returns to step 722 and continues through the remainder of the method.

Though aspects of the disclosure, particularly method 600 and portions of method 700, are described herein with respect to application of an invasive tool into the blood vessel to apply a palpation or perturbation, the instant disclosure is not so limited. Indeed, in accordance with the disclosure, the image processing of methods 300 and 600 may be made in combination with one or more additional or alternative stimuli to assess whether a patient is likely to be a "Responder" to a denervation therapy (i.e., expressing stiff blood vessels as a result of overstimulation of the sympathetic nerves along a blood vessel). In this aspect of the disclosure, a responder might be identified by achieving a pre-defined response to an invasive or non-invasive perturbation. Patients achieving the expected response to perturbation (depending on the nature of the perturbation e.g., application of vasodilator vs. vasoconstrictor, and accounting for any plaque or calcium build up on the artery wall) would be assumed to have higher sympathetic activity and thus be good candidates (e.g., Responders). As an example, a candidate for a denervation procedure may be required to demonstrate the capacity for vasodilation (e.g., of the renal artery or arterioles). If the vessels in the kidney are already generally dilated, for example by showing little to no response to application of a vasodilator, but showing a robust response to a vasoconstrictor, then the SNS may not be an important contributor to hypertension and therefore reducing SNS activity via a denervation procedure might be of minimal value. Similarly, if the renal arterioles have lost vascular function, and show no or little response to application of a vasodilator or a vasoconstrictor, (perhaps due to disease/calcification or other reasons) that too may indicate that reducing SNS activity via a denervation procedure might be of minimal value. A candidate that presents a constricted blood vessel and shows little to no response to a vasoconstrictor but a robust response to a vasodilator shows a likelihood that SNS activity plays a significant role in the hypertension of the patient and thus is a good candidate for denervation therapy. Finally, where both a response to vasodilators and vasoconstrictors is observed in the blood vessel (e.g., normal vascular function) the indication may be that denervation may have only a possible positive effect, or at minimum that denervation will have positive results is uncertain.

Non-invasive perturbations could include non-invasive stimulus such as Valsalva maneuver to slow heart rate and therewith the blood pressure of a patient, Müller's maneuver that decreases blood pressure during the maneuver and rapidly increases the blood pressure following the maneuver, cold pressor or mental stress (e.g., Stroop color test) that increases the blood pressure of a patient. An invasive stimulus could include intra-venous application of a vasodilator (NTG, adenosine, SNP, papaverine etc.) or a vasoconstrictor to lower or raise the blood pressure of the patient respectively. Or combinations of these stimuli.

Employing these alternative or additional stimuli, a clinician can undertake for example method 300 and acquire fluoroscopic images before application of the stimuli and after application of the stimuli to assess the impact on the change in blood pressure or the drugs on the measured stiffness and in connection with the method 700 identify whether a patient is a "Responder" or "Non-responder." The use of the two different measurements before and after stimulation provide a basis for normalizing the stiffness of the blood vessel based on the relative change in the distension of the blood vessel pre- and post-perturbation.

A further aspect of the disclosure is directed to the assessment of the efficacy of therapy as part of method 700. Efficacy can be assessed, for example, by application of a vasoconstrictor or vasodilator both before and after the denervation therapy. A blood vessel proximate highly stimulated sympathetic nerves will have little or no response to either drug owing to the blood vessel's already high stiffness. However, following a denervation procedure, where the nerves have been severed and thus the stiffness of the blood vessel has decreased, dosing with either a vasoconstrictor or a vasodilator may be expected to yield a measurable change in the diameter of the blood vessel. Thus, measurement of such changes and the comparison of these changes to those observed other patients can be employed to determine efficacy of the therapy and to quantify that efficacy as compared to a greater population. However, one of ordinary skill in the art will recognize that in patients with higher blood pressure there may be a more muted response to a vasodilator. Equally a vessel that has been sensitized by device manipulation, as described herein, may already be in a constricted state.

In accordance with the disclosure, peri-procedural measurements include repeated measurements of distensibility in the same patient before, during and after the procedure. Procedural success can be defined based on pre-treatment distensibility or stiffness reading consistent with that of a "Responder" and a post-procedure distensibility reading consistent with that of "Non-responder," for example. Still further, in accordance with the method 700 the denervation procedure can be terminated based on a peri-procedural reading that the blood vessel has transitioned from stiff to flexible crossing a predetermined threshold of distensibility. That threshold of distensibility can be based on population studies, as described above, and different threshold values of change of distensibility or stiffness can be determined for different categories of patients. For example, sex, weight, height, comorbidities may all be factor in the determination of the threshold change in stiffness or distensibility that is associated with a successful therapy and thus define different threshold or predetermined values that can be equated with a successful denervation. Regardless of the threshold for a specific patient set, an acute change in vessel mechanical properties following denervation is understood to be due to the loss of sympathetically mediated smooth muscle tone brought about by the denervation.

A further aspect of the disclosure is directed to assessing the stiffness of a blood vessel such as the renal artery along its length for example using method 300 or 600. However, rather than average the stiffness along the artery, the stiffness determinations can be made point wise (e.g., separately at P1 and P2) to determine whether there are portions of the blood vessel which exhibit greater stiffness than others. Locations of the blood vessel that exhibit noticeably greater stiffness than other portions of the blood vessel can then be targeted for denervation to further improve the likelihood of a positive outcome of the denervation procedure.

Though a number of outcomes are described herein above relating to the change in stiffness and a change in blood pressure the application is not so limited and other indicia of a successful outcome of a procedure may also be experienced and expected. As an example, for a patient with a pathologic SNS that is normotensive, but who nonetheless suffers from heart disease or any other disease that is associated with increased SNS activity, a successful denervation may result in improvements in heart failure symptoms such as a reduction in left ventricular mass. Thus, though described herein above as focused on hypertensive patient, other maladies may be addressed therapeutically using the methods and systems described herein for renal and other vasculature or body lumen denervation.

As will be appreciated herein, though described as a threshold of stiffness for identifying a "Responder" and a "Non-responder" as well as a threshold change in stiffness for determining the success of a denervation procedure, the disclosure is not so limited and a range or continuum of values of stiffness and can be utilized without departing from the scope of the disclosure to identify "Responders" and "Non-responders" and to assess the efficacy of the therapy.

Though varies examples are described and illustrated herein that employ fluoroscopic imaging, this disclosure is not so limited, and any medical imaging technique may be used to observe the blood vessels to undertake the analyses described herein. Imaging methodologies include ultrasound, magnetic resonance imaging (MRI), computed tomography (CT) , cone beam computed tomography (CBCT), optical coherence tomography (OCT), and others, both known now, and those future developed modalities without departing from the scope of the disclosure.

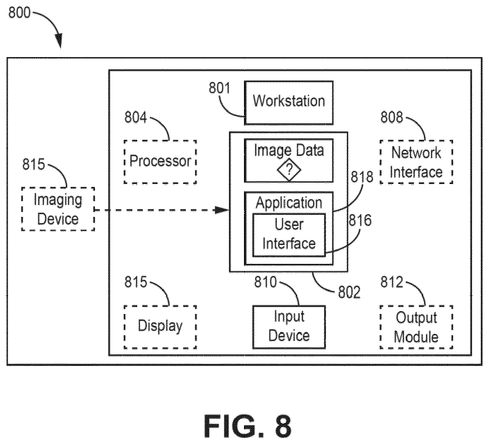

Reference is now made to FIG. 8, which is a schematic diagram of a system 800 configured for use with the methods of the disclosure including the methods 300, 600, 700. System 800 may include a workstation 801. Workstation 801 may include a memory 802, a processor 804, a display 806 and an input device 810. Processor or hardware processor 804 may include one or more hardware processors. Workstation 801 may optionally include an output module 812 and a network interface 808. Memory 802 may store an application 818 and image data 814. Application 818 may include instructions executable by processor 804 for executing the methods of the disclosure including the methods 300, 600, 700 and other described in conjunction therewith.

Application 818 may further include a user interface 816. Image data 814 may include the computed tomography (CT) scans, fluoroscopic images, fluoroscopic 3D reconstructions, ultrasound data, OCT image data and others. Processor 804 may be coupled with memory 802, display 806, input device 810, output module 812, network interface 808 and imaging device 815. In some embodiments, workstation 801 may be coupled with imaging device 815 (e.g., a fluoroscope, MRI imager, CT imager, ultrasound imager, or others), directly or indirectly, e.g., by wireless communication. Workstation 801 may be a stationary computing device, such as a personal computer, or a portable computing device such laptop or tablet computer.

Memory 802 may include any non-transitory computer-readable storage media for storing data and/or software including instructions that are executable by processor 804 and which control the operation of workstation 801 and, in some embodiments, may also control the operation of imaging device 815. Imaging device 815 may be used to capture a sequence of images as described above in connection with methods 300, 600, 700. In an aspect of the disclosure, memory 802 may include one or more storage devices such as solid-state storage devices, e.g., flash memory chips. Alternatively, or in addition to the one or more solid-state storage devices, memory 802 may include one or more mass storage devices connected to the processor 804 through a mass storage controller (not shown) and a communications bus (not shown).

Although the description of computer-readable media contained herein refers to solid-state storage, it should be appreciated by those skilled in the art that computer-readable storage media can be any available media that can be accessed by the processor 804. That is, computer readable storage media may include non-transitory, volatile and non-volatile, removable and non-removable media implemented in any method or technology for storage of information such as computer-readable instructions, data structures, program modules or other data. For example, computer-readable storage media may include RAM, ROM, EPROM, EEPROM, flash memory or other solid-state memory technology, CD-ROM, DVD, Blu-Ray or other optical storage, magnetic cassettes, magnetic tape, magnetic disk storage or other magnetic storage devices, or any other medium which may be used to store the desired information, and which may be accessed by workstation 801.

Application 818 may, when executed by processor 804, cause display 806 to present user interface 816. User interface 816 may be configured to present to the user a single screen including a live view of images, showing the relevant blood vessels as described herein.

Network interface 808 may be configured to connect to a network such as a local area network (LAN) consisting of a wired network and/or a wireless network, a wide area network (WAN), a wireless mobile network, a Bluetooth network, and/or the Internet. Network interface 808 may be used to connect between workstation 801 and imaging device 815. Network interface 808 may be also used to receive image data 814. Input device 810 may be any device by which a user may interact with workstation 801, such as, for example, a mouse, keyboard, foot pedal, touch screen, and/or voice interface. Output module 812 may include any connectivity port or bus, such as, for example, parallel ports, serial ports, universal serial busses (USB), or any other similar connectivity port known to those skilled in the art.

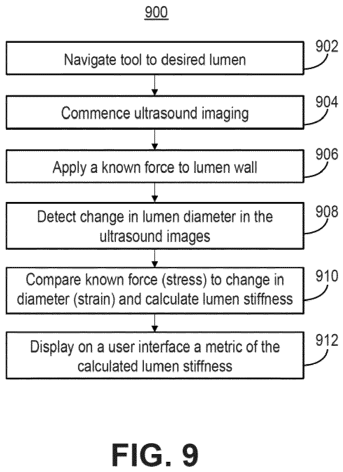

Fig. 9 depicts a further method 900 in accordance with the disclosure. Method 900 is directed to a method employing ultrasound strain elastography (i.e., acoustic imaging). Ultrasound strain elastography is a method of measuring tissue stiffness often used for assessing liver disease or breast tumors. However, known methodologies only provide a relative stiffness of tissue. In contrast, by employing a denervation tool 402 or palpitation device, as described herein above, a known force can be applied to a body lumen wall. Ultrasound imaging is employed to detect a deflection of the body lumen wall by the application of the known force. By comparing the known force, a stress, to the displacement of the body lumen wall, a strain, a quantitative stiffness value can be calculated.

Method 900 starts with the navigation of a denervation tool 402 or palpitation tool to a desired location within a body lumen (e.g., within the renal or hepatic artery). At step 904 ultrasound imaging is commenced. A known force is applied to the body lumen wall at step 906 using the denervation tool 402 or a palpitation tool. Using, for example, image processing software a change in body lumen diameter caused by the application of the known force is detected at step 908. At step 910 a comparison of the known force (stress) to the displacement (strain) of the body lumen wall is undertaken and a body lumen wall (e.g., renal artery or hepatic artery) stiffness is calculated. As with other methods, at step 912 a metric of the calculated body lumen stiffness can be displayed. This metric may be an indication that the patient is determined to be a "Responder" or not, a value of body lumen wall stiffness, or another indicator as described elsewhere herein.

While several aspects of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular aspects.

Further disclosed herein is the subject-matter of the following clauses:
1. A method of determining blood vessel stiffness comprising:
   acquiring a plurality of images of a portion of a blood vessel;
   analyzing the plurality of images to determine a maximum diameter of the portion of the blood vessel at a plurality of points along the blood vessel;
   analyzing the plurality of images to determine a minimum diameter of the portion of the blood vessel at the plurality of points along the blood vessel;
      determining a blood pressure experienced in the portion of the blood vessel; and
   calculating a stiffness of the portion of the blood vessel based on a ratio of the blood pressure and a difference in a diameter of the blood vessel at one or more of the plurality of points along the blood vessel.
2. The method of clause 1, further comprising calculating a pulse wave velocity of blood in the portion of the blood vessel based on a difference in time at which a maximum diameter is reached at two or more of the plurality of points along the blood vessel.
3. The method of clause 1, further comprising identifying the plurality of points along the blood vessel;
   defining at each point a vector normal to a wall of the blood vessel; and
   measuring the maximum and minimum diameters along the vector between the point and an opposing point at which the vector intersects an opposite wall of the blood vessel.
4. The method of clause 1, further comprising denervating at the portion of the blood vessel.
5. The method of clause 4, further comprising calculating a second stiffness of the portion of the blood vessel following the denervation, wherein a reduction in stiffness in excess of a threshold value is indicative of a successful denervation.
6. The method of clause 5, wherein the portion of the blood vessel is a portion of a renal artery, a hepatic artery, a splanchnic artery, a celiac artery, a superior mesenteric artery, or an inferior mesenteric artery, and successful denervation results in a drop in the blood pressure of a patient.
7. The method of clause 5, wherein the second stiffness is calculated peri-procedurally.
8. The method of clause 1, wherein portion of the blood vessel is a portion of a renal artery of a patient, and the calculated stiffness of the renal artery is indicative of a sympathetic nerve activity along the renal artery.
9. A method of assessing a stiffness of a blood vessel comprising:
   inserting an elongate tool into a blood vessel;
   applying a known force to a wall of the blood vessel using the elongate tool;
   capturing a plurality of images of the blood vessel;
   measuring in the plurality of images a change in diameter of the blood vessel caused by application of the known force by the elongate tool to the wall of the blood vessel; and
   calculating a stiffness of the blood vessel based on a ratio of the known force and the measured change in diameter of the blood vessel
10. The method of clause 9, wherein the blood vessel is a portion of a renal artery of a patient, and the calculated stiffness of the renal artery is indicative of a sympathetic nerve activity along the renal artery.
11. The method of clause 10, wherein the elongate tool is a denervation tool.
12. The method of clause 10, further comprising denervating at the renal artery.
13. The method of clause 11, further comprising calculating a second stiffness of the renal artery, wherein a reduction in stiffness in excess of a threshold value is indicative of a successful denervation.
14. The method of clause 13, wherein the second stiffness is calculated peri-procedurally.
15. A method of assessing stiffness of a blood vessel, comprising:
   measuring a blood pressure of a patient;
   calculating a stiffness of a blood vessel identified for receiving denervation therapy;
   normalizing the calculated stiffness of the blood vessel relative to the measured blood pressure of the patient; and
   determining that the patient is likely to experience a positive response to a denervation when the normalized stiffness is greater than a predetermined value.
16. The method of clause 15, further comprising measuring a mean aortic pressure (MAP) of the patient and comparing the measured MAP and calculated stiffness to a database of MAP and stiffness data to determine the normalized stiffness of the blood vessel.
17. The method of clause 15, further comprising comparing the calculated stiffness and measured blood pressure of the blood vessel to known tissue properties at a reference blood pressure to determine the normalized stiffness of the blood vessel.
18. The method of clause 15, further comprising:
   measuring a blood pressure of a plurality of patients;
   calculating a stiffness of a blood vessel for the plurality of patients; and
   correlating the measured blood pressure and calculated blood vessel stiffness of the plurality of patients to an expected outcome of denervation of the blood vessel.
19. The method of clause 15, wherein normalizing the calculated stiffness further comprises applying a stimulus to the patient and calculating an after-stimulus stiffness of the blood vessel, wherein a response to the stimulus is indicative of a likelihood of a patient experiencing a positive response to a denervation of the blood vessel.
20. The method of clause 19, wherein the stimulus is a non-invasive stimulus including one or more of a Valsalva maneuver, Müller's maneuver, cold pressor, or a mental stress test.
21. A system for determining blood vessel stiffness comprising:
   means for acquiring a plurality of images of a portion of a blood vessel;
   means for analyzing the plurality of images to determine a maximum diameter of the portion of the blood vessel at a plurality of points along the blood vessel;
   means for analyzing the plurality of images to determine a minimum diameter of the portion of the blood vessel at the plurality of points along the blood vessel;
   means for determining a blood pressure experienced in the portion of the blood vessel;
      and
   means calculating a stiffness of the portion of the blood vessel based on a ratio of the blood pressure and a difference in a diameter of the blood vessel at one or more of the plurality of points along the blood vessel.
22. The system of clause 21, further comprising means for calculating a pulse wave velocity of blood in the portion of the blood vessel based on a difference in time at which a maximum diameter is reached at two or more of the plurality of points along the blood vessel.
23. The system of clause 21, further comprising means for identifying the plurality of points along the blood vessel;
   means for defining at each point a vector normal to a wall of the blood vessel; and
   means for measuring the maximum and minimum diameters along the vector between the point and an opposing point at which the vector intersects an opposite wall of the blood vessel.
24. The system of clause 21, further comprising means for denervating at the portion of the blood vessel.
25. The system of clause 24, further comprising means for calculating a second stiffness of the portion of the blood vessel following the denervation, wherein a reduction in stiffness in excess of a threshold value is indicative of a successful denervation.
26. The system of clause 25, wherein the portion of the blood vessel is a portion of a renal artery, a hepatic artery, a splanchnic artery, a celiac artery, a superior mesenteric artery, or an inferior mesenteric artery, and successful denervation results in a drop in the blood pressure of a patient.
27. The system of clause 25, wherein the second stiffness is calculated peri-procedurally.
28. The system of clause 21, wherein the portion of the blood vessel is a portion of a renal artery of a patient, and the calculated stiffness of the renal artery is indicative of a sympathetic nerve activity along the renal artery.
29. A system configured for assessing a stiffness of a blood vessel comprising:
   an elongate tool configured for being inserted into a blood vessel and configured for applying a known force to a wall of the blood vessel;
   means for capturing a plurality of images of the blood vessel;
   means for measuring in the plurality of images a change in diameter of the blood vessel caused by application of the known force by the elongate tool to the wall of the blood vessel; and
   means for calculating a stiffness of the blood vessel based on a ratio of the known force and the measured change in diameter of the blood vessel
30. The system of claim 29, wherein the blood vessel is a portion of a renal artery of a patient, and the calculated stiffness of the renal artery is indicative of a sympathetic nerve activity along the renal artery.
31. The system of claim 30, wherein the elongate tool is a denervation tool.
32. The system of claim 31, further comprising means for calculating a second stiffness of the renal artery, wherein a reduction in stiffness in excess of a threshold value is indicative of a successful denervation.
33. The system of claim 32, wherein the second stiffness is calculated peri-procedurally.
34. A system for assessing stiffness of a blood vessel, comprising:
   means for measuring a blood pressure of a patient;
   means for calculating a stiffness of a blood vessel identified for receiving denervation therapy;
   means for normalizing the calculated stiffness of the blood vessel relative to the measured blood pressure of the patient; and
   means for determining that the patient is likely to experience a positive response to a denervation when the normalized stiffness is greater than a predetermined value.
35. The system of clause 34, further comprising means for measuring a mean aortic pressure (MAP) of the patient and for comparing the measured MAP and calculated stiffness to a database of MAP and stiffness data to determine the normalized stiffness of the blood vessel.
36. The system of clause 34, further comprising means for comparing the calculated stiffness and measured blood pressure of the blood vessel to known tissue properties at a reference blood pressure to determine the normalized stiffness of the blood vessel.
37. The system of clause 34, further comprising:
   means for measuring a blood pressure of a plurality of patients;
   means for calculating a stiffness of a blood vessel for the plurality of patients and for correlating the measured blood pressure and calculated blood vessel stiffness of the plurality of patients to an expected outcome of denervation of the blood vessel.
38. The system of clause 34, wherein system further comprises means for applying a stimulus to the patient and wherein the means for normalizing the calculated stiffness are further configured for calculating an after-stimulus stiffness of the blood vessel, wherein a response to the stimulus is indicative of a likelihood of a patient experiencing a positive response to a denervation of the blood vessel.
39. The system of clause 38, wherein the stimulus is a non-invasive stimulus including one or more of a Valsalva maneuver, Müller's maneuver, cold pressor, or a mental stress test.

## Claims

1. A method of determining blood vessel stiffness comprising:
acquiring a plurality of images of a portion of a blood vessel;
analyzing the plurality of images to determine a maximum diameter of the portion of the blood vessel at a plurality of points along the blood vessel;
analyzing the plurality of images to determine a minimum diameter of the portion of the blood vessel at the plurality of points along the blood vessel;
determining a blood pressure experienced in the portion of the blood vessel; and
calculating a stiffness of the portion of the blood vessel based on a ratio of the blood pressure and a difference in a diameter of the blood vessel at one or more of the plurality of points along the blood vessel.

2. The method of claim 1, further comprising calculating a pulse wave velocity of blood in the portion of the blood vessel based on a difference in time at which a maximum diameter is reached at two or more of the plurality of points along the blood vessel.

3. The method of claim 1, further comprising
identifying the plurality of points along the blood vessel;
defining at each point a vector normal to a wall of the blood vessel; and
measuring the maximum and minimum diameters along the vector between the point and an opposing point at which the vector intersects an opposite wall of the blood vessel.

4. The method of claim 3, further comprising calculating a second stiffness of the portion of the blood vessel following denervation at the portion of the blood vessel, wherein a reduction in stiffness in excess of a threshold value is indicative of a successful denervation.

5. The method of claim 4, wherein the portion of the blood vessel is a portion of a renal artery, a hepatic artery, a splanchnic artery, a celiac artery, a superior mesenteric artery, or an inferior mesenteric artery, and successful denervation results in a drop in the blood pressure of a patient.

6. The method of claim 4, wherein the second stiffness is calculated peri-procedurally.

7. A system configured for assessing a stiffness of a blood vessel comprising:
an elongate tool configured for being inserted into a blood vessel and configured for applying a known force to a wall of the blood vessel;
means for capturing a plurality of images of the blood vessel;
means for measuring in the plurality of images a change in diameter of the blood vessel caused by application of the known force by the elongate tool to the wall of the blood vessel; and
means for calculating a stiffness of the blood vessel based on a ratio of the known force and the measured change in diameter of the blood vessel

8. The system of claim 7, wherein the blood vessel is a portion of a renal artery of a patient, and the calculated stiffness of the renal artery is indicative of a sympathetic nerve activity along the renal artery.

9. The system of claim 8, wherein the elongate tool is a denervation tool.

10. The system of claim 9, further comprising means for calculating a second stiffness of the renal artery, wherein a reduction in stiffness in excess of a threshold value is indicative of a successful denervation.

11. The system of claim 10, wherein the second stiffness is calculated peri-procedurally.

12. A method of assessing stiffness of a blood vessel, comprising:
measuring a blood pressure of a patient;
calculating a stiffness of a blood vessel identified for receiving denervation therapy;
normalizing the calculated stiffness of the blood vessel relative to the measured blood pressure of the patient; and
determining that the patient is likely to experience a positive response to a denervation when the normalized stiffness is greater than a predetermined value.

13. The method of claim 12, further comprising measuring a mean aortic pressure (MAP) of the patient and comparing the measured MAP and calculated stiffness to a database of MAP and stiffness data to determine the normalized stiffness of the blood vessel.

14. The method of claim 12, further comprising comparing the calculated stiffness and measured blood pressure of the blood vessel to known tissue properties at a reference blood pressure to determine the normalized stiffness of the blood vessel.

15. The method of claim 12, further comprising:
measuring a blood pressure of a plurality of patients;
calculating a stiffness of a blood vessel for the plurality of patients; and
correlating the measured blood pressure and calculated blood vessel stiffness of the plurality of patients to an expected outcome of denervation of the blood vessel.

16. The method of claim 12, wherein normalizing the calculated stiffness further comprises applying a stimulus to the patient and calculating an after-stimulus stiffness of the blood vessel, wherein a response to the stimulus is indicative of a likelihood of a patient experiencing a positive response to a denervation of the blood vessel.
